# EUROPÄISCHE PATENTSCHRIFT

(11) **EP 1 251 165 B1**
(45) Veröffentlichungstag und Bekanntmachung des Hinweises auf die Patenterteilung: **02.04.2008**
(21) Anmeldenummer: 01117917.3
(22) Anmeldetag: 24.07.2001
(51) Int. Cl.: C12M 1/107

(54) **Biomasse-Zudosier-Vorrichtung an einem Fermenter einer Biogasanlage**
Device for addition of biomass to a fermenter making part of biogas plant
Appareil pour le dosage de biomasse à un fermenteur faisant part d'une installation de biogaz

(30) Priorität: 19.04.2001 DE 20106837 U
(43) Veröffentlichungstag der Anmeldung: 23.10.2002
(73) Patentinhaber: U.T.S. Umwelt-Technik-Süd GmbH, 84419 Obertaufkirchen (DE)
(72) Erfinder: Bürger, Adam, 84405 Grüntegernbach (DE)
(74) Vertreter: Liebl, Thomas

(56) Entgegenhaltungen:
- DE-C- 19 732 198
- DE-U- 29 820 752
- DE-U- 29 903 208
- FR-A- 2 673 853
- FR-A- 2 772 641

## Beschreibung

Die Erfindung betrifft eine Biomasse-Zudosier-Vorrichtung an einem Fermenter einer Biogasanlage nach dem Oberbegriff des Anspruchs 1.

Ein Fermenter zur anaeroben Fermentation von organischen Stoffen einer Biogasanlage ist beispielsweise aus der DE 196 21 914 C1 bekannt. Bei bekannten Anlagen wird vorrangig Flüssigmist/Gülle fermentiert, die dem Fermenter über Pumpsysteme und Rohrleitungen zugeführt werden.

Es ist ferner auch allgemein bekannt, organische Flüssigkeiten und/oder Feststoffe als Biomasse, wie beispielsweise Wirtschaftsdünger aus der Landwirtschaft (Rindergülle, Rinder-Festmist, Schweinegülle, Schweine-Festmist, Hühnergülle, Hühner-Trockenkot) und/oder landwirtschaftliche Reststoffe (Grasschnitt-Rübenblätter, Silagen) und/oder Reststoffe aus der Agroindustrie oder verwandten Industrien (Biertreber, Obsttrester, Gemüsereste, Rapsschrot, Getreideabputz, Schlempen, Melasse) oder dergleichen beim Fermentationsvorgang zuzudosieren. Diese organischen Feststoffe sind somit oftmals keine reinen Feststoffe, sondern mehr oder weniger mit einer Flüssigkeit vermischte Feststoffe.

Eine Zudosierung erfolgt in an sich bekannter Weise über einen verschließbaren Fermenterbehälter-Einwurfschacht, der in der Art eines Tauchrohrs bis unter den Flüssigkeitsspiegel des Fermenters geführt ist. Derartige Biogasanlagen finden meist in der dezentralen Verarbeitung und Verwertung von organischen Feststoffen im landwirtschaftlichen Bereich Verwendung, insbesondere in landwirtschaftlichen Großbetrieben, die die organischen Feststoffe direkt vor Ort verwerten und als Energie nutzen. Dazu werden die im Fermenter erzeugten Biogase in nachgeschalteten Brennkraftmaschinen mit Elektrogeneratoren zur Stromerzeugung verbrannt. Dieser so erzeugte Strom kann dann vor Ort in das Stromnetz eingespeist werden. Zur Beschickung der Fermenterbehälter mit Biomasse wird diese von den Landwirten mittels herkömmlicher Transportwagen, z. B. einem Mistwagen, einem Güllewagen oder dergleichen, in den Bereich des Einwurfschachts gefahren und anschließend die sich im Transportwagen befindliche Menge über den Einwurfschacht in den Fermenterbehälter eingeworfen bzw. eingelassen. Alternativ dazu ist es auch bekannt, die Biomasse aus Gruben in den Fermenterbehälter zu pumpen.

Insbesondere bei der Zuführung von größeren Mengen an Biomasse ergeben sich hier Probleme, da diese neu eingebrachte Biomasse aufschwimmen oder plötzlich absacken kann. Weiter ergibt sich aufgrund der unkontrollierten Zugabe insbesondere von größeren Mengen an Biomasse auch eine plötzliche Veränderung der Gegebenheiten im Fermenter selbst, die regelmäßig zu einer erhöhten Beanspruchung der höchstempfindlichen Bakterienkulturen führt, da diese dann eine erhöhte Fermentationsarbeit leisten müssen, was wiederum zu Temperaturschwankungen im Fermenterinneren führt. Diese Temperaturschwankungen wirken sich regelmäßig nachteilig auf die Bakterienkulturen selbst aus, die durch einen plötzlichen Temperaturanstieg beeinträchtigt oder sogar zerstört werden können. Dadurch kann ggf. der gesamte Fermentationsprozess zusammenbrechen. Weiter bewirkt ein Temperaturanstieg von bereits wenigen Grad Celsius im Fermenterbehälter einen höheren Druck, was wiederum zu Rissen in den sowieso schon hochbeanspruchten, regelmäßig aus Beton hergestellten Fermenterwänden, insbesondere z. B. in den Übergangsbereichen zwischen Seitenwand und Deckenwand, führen kann. Unter Umständen kann hierdurch sogar die Decke von den oder der Seitenwand abgehoben werden.

Weiter wird durch eine derartige unkontrollierte Zugabe, insbesondere auch bei einer Zugabe von größeren Mengen Biomasse, was regelmäßig der Fall ist, nachteiligerweise auch keine Erhöhung der Gasausbeute erreicht, sondern je nach Art und Menge der zugegebenen Biomasse unter Umständen sogar eine Verschlechterung des Gasausbeute-Wirkungsgrades erreicht.

Aus der gattungsgemäßen DE 200 11 785 U1 ist bereits eine Biomasse-Zudosier-Vorrichtung an einem Fermenter einer Biogasanlage bekannt, die einen Fermenterbehälter aufweist, der wiederum wenigstens eine Fermenterzuführöffnung aufweist, über die organische Feststoffe als Biomasse chargenweise in den Fermenterbehälter einbringbar sind.

Konkret besteht die Biomasse-Zudosier-Vorrichtung aus einem Einwurfschacht mit anschließendem Zwischensammelraum für organische Feststoffe und einem nachgeordneten Förderrohr sowie einer aus einer Kolbenpresse bestehenden Linearfördereinrichtung. Der Pressenstempel der Kolbenpresse ist im Bereich des Zwischensammelraums angetrieben bewegbar, so dass für eine chargenweise Förderung im Zwischensammelraum enthaltene organische Feststoffe mittels des Pressenstempels in und durch das Förderrohr pressbar sind. Bei zurückgezogenem Pressenstempel kann somit der Zwischensammelraum und ggfs. der damit verbundene Einwurfschacht mit organischen Feststoffen gefüllt werden. Anschließend kann der Pressenstempel durch den Zwischensammelraum geschoben werden, wodurch dieser ausgeräumt wird und die darin vorher enthaltenen organischen Feststoffe in das Förderrohr gepresst werden. Der Zwischensammelraum wirkt dabei in der Art eines Zylinderraums für den Pressenstempel. Das Förderrohr ist zudem dicht mit einer Fermenterzuführöffnung verbunden, so dass die geförderten organischen Feststoffe in den Fermenterbehälter zuführbar sind, ohne dass Biogas aus dem Fermenterbehälter durch den Förderrohranschluss und um diesen herum austreten kann.

Bei einer derartigen chargenweisen Zudosierung wird zwar pro Kolbenhub nur eine bestimmte Feststoffmenge in den Fermenterbehälter eingebracht, wobei es jedoch auch hier je nach der Anzahl der durchgeführten Kolbenhübe und damit der innerhalb relativ kurzer Zeit unkontrolliert in den Fermenterbehälter zudosierten Biomassemenge zu einem Aufschwimmen von Zudosierstoffen oder zu einem plötzlichen Absacken von Biomasse und damit zu einer plötzlichen Veränderung der Gegebenheiten im Fermenter selbst kommen kann, was wiederum zu einer erhöhten Beanspruchung der höchst empfindlichen Bakterienkulturen führt. Diese erhöhte Beanspruchung der Bakterienkulturen infolge einer erhöhten zu leistenden Fermentationsarbeit kann wiederum in unerwünschter Weise zu Temperaturschwankungen im Fermenterinneren führen. Dies wirkt sich regelmäßig nachteilig auf die Bakterienkulturen aus, die durch einen plötzlichen Temperaturanstieg beeinträchtigt oder sogar komplett zerstört werden können, so dass unter Umständen sogar der gesamte Fermententationsprozess zusammenbrechen kann. Ein derartiger Temperaturanstieg von bereits wenigen Grad Celsius im Fermenterbehälter kann einen höheren Druck bewirken, was wiederum zu den bereits weiter oben ausführlich beschriebenen nachteiligen Folgen für den Fermenterbehälter führen kann. Des weiteren wird auch hier durch eine derartige unkontrollierte Zugabe oftmals keine Erhöhung der Gasausbeute erreicht, sondern je nach Art und Menge der zugegebenen Biomasse unter Umständen sogar eine Verschlechterung des Wirkungsgrades der Biogasausbeute erreicht. Ein weiterer Nachteil dieses Aufbaus ist, dass sich das Förderrohr insbesondere bei längerer Nichtbetätigung des Pressenstempels so zusetzen kann, dass dieses mit dem Pressenstempel nicht mehr freigeräumt werden kann.

Eine andere Problematik betrifft die DE 298 20 752 U1, die den Wirkungsgrad einer Biogasanlage dadurch erhöhen will, dass in einem dem Fermenterbehälter vorgeschalteten Mischbehälter eine flüssige Biomasse-Mischung mit genau definierter Zusammensetzung erzeugt wird, die anschließend in den Fermenterbehälter gepumpt wird. Zur Erzeugung dieser optimalen Mischung ist der Mischbehälter auf einer Waage angeordnet, so dass die zu mischenden Komponenten Gülle und die weiteren Zusatzstoffe mengenmäßig genau in den Mischbehälter eindosiert werden können. In dem Mischbehälter selbst sind Mischereinrichtungen angeordnet, die die in den Mischbehälter eingebrachte Gülle und die weiteren Zusatzstoffe so vermischen, dass sich ein homogenes Gemisch bildet. Die so mittels der Wägeeinrichtung exakt zusammenstellbare Biomasse-Mischung wird dann in üblicher Weise mittels einer Pumpeinrichtung ggf. unter Zwischenschaltung eines Hygienisierers in einem seitlichen Fermenterbereich unterhalb der Gasphase zugeführt. Aufgrund des hohen Bestandteils an Gülle ist diese Biomasse-Mischung sehr flüssig und daher erfolgt die Zuführung zum Fermenter im wesentlichen kontinuierlich durch Pumpen. Bei derartigen flüssigen Biomasse-Mischungen tritt die zuvor genannte Problematik des Aufschwimmens bzw. plötzlichen Absackens von Biomasse-Feststoffen nicht auf. Die hier beschriebene Verfahrensführung ist somit insbesondere für die Erzeugung von Biogas aus im wesentlichen flüssiger Biomasse geeignet und für solche Biogasanlagen, bei denen ein großer Anteil von organischen Feststoffen als Biomasse vorhanden ist, nicht geeignet. Insbesondere ist diese Verfahrensführung nicht geeignet für sehr groß bauende Biogasanlagen, bei denen die Biomasse in Form von Feststoffen mit z. B. Lastwägen in sehr großen Mengen angeliefert wird und in solchen Vorratsbehältern gespeichert wird, deren Volumen ein Vielfaches einer Lastwagenladung beträgt, sowie bei denen sehr große Feststoffchargen in den Fermenterbehälter zudosiert werden. Ein weiteres Problem einer derartigen Verfahrensführung ist, dass der Einsatz von Mischeinrichtungen, wie z. B. Rührflügeln, in einem dem Fermenter vorgeschalteten Mischbehälter zusätzlich zu den ohnehin im Fermenterbehälter vorhandenen Rührelementen einen großen energetischen Aufwand erfordern, was wiederum den Gesamtwirkungsgrad der Biogasanlage reduziert. Insbesondere bei den eben erwähnten sehr groß bauenden Biogasanlagen, die mit großen Feststoffmengen beschickt werden, ist eine derartige Vermischung mittels Mischeinrichtungen auch technisch nur sehr schwer zu realisieren.

Demgegenüber ist es Aufgabe der Erfindung, eine Biomasse-Zudosier-Vorrichtung an einem Fermenter einer Biogasanlage zu schaffen, mit der eine Zudosierung von Biomasse-Feststoffen in den Fermenterbehälter hinein so durchführbar ist, dass es im Fermenterbehälter zu keinem unkontrolliertem Aufschwimmen oder plötzlichem Absacken von zudosierter Biomasse und damit zu keiner erhöhten Beanspruchung der Bakterienkulturen bzw. zu keiner für die Bakterienkulturen und/oder den Fermenterbehälter selbst nachteiligen Temperaturerhöhung im Fermenterbehälterinnenraum kommen kann.

Diese Aufgabe wird gelöst mit den Merkmalen des Anspruchs 1.

Gemäß Anspruch 1 weist die Biomasse-Zudosier-Vorrichtung Mittel auf, mit denen eine genau auf den Fermentationsprozess abgestimmte Biomassemenge chargenweise in den Fermenterbehälter einbringbar ist, wobei die Mittel durch eine Wägeeinrichtung als Gewichtsmesseinrichtung gebildet sind.

Mit derartigen als Wägeeinrichtung ausgebildeten Mitteln kann eine genau auf die Fermentationsgegebenheiten abgestimmte Biomassemenge in den Fermenterbehälter eingebracht werden, was sich vorteilhaft auf den gesamten Fermentationsprozess und damit auf den Wirkungsgrad der Gasausbeute auswirkt. Eine Vermischung der Biomasse erfolgt hier dann im Fermenterbehälter selbst, und zwar durch den Einsatz dort angeordneter Rührelemente. Mit einer derartigen, genau abstimmten und in den Fermenterbehälter eingebrachten Biomassemenge kann nämlich auf einfache Weise ein Aufschwimmen von Zudosierstoffen oder ein mögliches, plötzliches Absacken und Eindringen von großen Zudosiermengen mit ungünstigen Auswirkungen auf den laufenden Fermentationsprozess sehr gut verhindert werden. Dadurch kann es bei einem derartigen, hinsichtlich der Menge der zudosierten Biomasse genau abgestimmten Fermentationsprozess zu keiner erhöhten Beanspruchung der Bakterienkulturen und damit zu keiner für die Bakterienkulturen und/oder den Fermenterbehälter selbst nachteiligen Temperaturerhöhung im Fermenterbehälterinnenraum kommen. Dadurch ist die Gefahr einer Beeinträchtigung der Bakterienkulturen sowie einer Beschädigung des Fermenterbehälters selbst wesentlich reduziert. Dadurch, dass die Mittel durch eine Wägeeinrichtung als Gewichtsmesseinrichtung gebildet sind, ist auf einfache, schnelle und preiswerte Weise bei der Zudosierung einer exakt festgelegten Biomassemenge in den Fermenterbehälter eine mengenmäßige Kontrolle sehr gut möglich.

Zudem wird mit einem derartigen Aufbau einer Biomasse-Zudosier-Vorrichtung auf einfache Weise auch ein weitgehend automatisierter Intervallbetrieb einer Biogasanlage möglich, bei der je nach den Erfordernissen, z. B. im Stunden- oder im Tagesabstand, bestimmte, genau abgewogene Biomassemengen in den Fermenterbehälter eingebracht werden können.

Grundsätzlich gibt es verschiedene Möglichkeiten, eine Wägeeinrichtung in Verbindung mit der Biomasse-Zudosier-Vorrichtung anzuordnen, so kann beispielsweise nach Anspruch 2 eine Wägeeinrichtung in Verbindung mit einem Förderband vorgesehen sein. Gemäß einer alternativen Ausgestaltung nach Anspruch 3 ist ein Vorratsbehälter vorgesehen, in dem die Biomasse aufgenommen ist, wobei eine Messeinrichtung, vorzugsweise eine Wägeeinrichtung, mit dem Vorratsbehälter gekoppelt ist. Im Falle einer Wägeeinrichtung kann diese dann z.B. auch die jeweils aktuell im Vorratsbehälter befindliche Biomassemenge anzeigen. Über einen derartigen Vorratsbehälter in Verbindung mit einer Messeinrichtung, vorzugsweise einer Wägeeinrichtung, kann somit das kontrollierte Einbringen einer genau abgestimmten Biomassemenge in den Fermenterbehälter auf besonders einfache Weise ohne großen Bauteilaufwand realisiert werden.

Weiter gibt es grundsätzlich verschiedene Möglichkeiten, die Wägeeinrichtung auszubilden. So kann diese beispielsweise unabhängig vom Vorratsbehälter im Aufstandsbereich desselben angeordnet sein. Gemäß einer besonders bevorzugten Ausführungsform nach Anspruch 4 ist die Wägeeinrichtung durch wenigstens einen Wägestab gebildet ist, auf dem der Vorratsbehälter gelagert ist und der vorzugsweise integral mit dem Vorratsbehälter ausgebildet ist. Eine derartige Wägeeinrichtung mit wenigstens einem Wägestab ist bei hoher Präzisionssicherheit einfach und preiswert an einem Vorratsbehälter vorzusehen. Zudem sind derartige Wägestäbe relativ unempfindlich und können daher auch im rauen landwirtschaftlichen Betrieb funktionssicher und langdauernd eingesetzt werden. Nach Anspruch 5 können hier mehrere Wägestäbe vorgesehen sein, mit denen bei ungleichmäßiger Biomasseverteilung im Vorratsbehälter ein gemittelter Biomasse-Gewichtswert ermittelbar ist. Dadurch wird die Funktionssicherheit der Wägeeinrichtung weiter wesentlich erhöht, da eventuelle Falschmessungen auf einfache Weise vermieden werden. Zudem kann die Anzeige der sich jeweils aktuell im Vorratsbehälter befindlichen Biomassenmenge über eine Anzeigeeinrichtung am Vorratsbehälter und damit unmittelbar vor Ort am Fermenter angezeigt werden. Alternativ dazu ist jedoch auch eine Anzeige über z. B. Bildschirme oder dergleichen vom Fermenter entfernt in einer Kontrollstation möglich, wobei die Daten dann über entsprechende Leitungen zu einer Auswerte- und/oder Steuereinrichtung einer Kontrollstation geleitet werden.

Gemäß einer konkreten Ausgestaltung nach Anspruch 6 ist im Vorratsbehälter wenigstens eine verschließbare Behälter-Auslassöffnung vorgesehen, über die im geöffneten Zustand eine vorzugsweise genau abgestimmte Biomassemenge freigebbar und über die Fermenterzuführöffnung in den Fermenterbehälter einbringbar ist. Gemäß einer ersten Ausführungsform hierzu ist nach Anspruch 7 vorgesehen, dass über die Behälter-Auslassöffnung eine genau abgestimmte Biomassemenge freigegeben wird, wobei ein Förderrohr gasdicht von der Behälter-Auslassöffnung ausgehend durch die Fermenterzuführöffnung geführt ist und mit einem Rohrende in die Fermenterflüssigkeit eintaucht. Durch dieses Eintauchen des Förderrohrendes in die Fermenterflüssigkeit wird vorteilhaft erreicht, dass kein Biogas, das sich oberhalb des Flüssigkeitsspiegels im Fermenterbehälter befindet aus dem Fermenterbehälter entweichen kann. Damit sind keine besonderen Abdichtmaßnahmen mehr erforderlich. Ein derartiges Förderrohr ist besonders einfach und preiswert herzustellen, so dass sich ein insgesamt preiswerter Aufbau einer derartigen Feststoff-Zudosier-Vorrichtung realisieren lässt. Gemäß einer besonders bevorzugten Weiterentwicklung hierzu kann sich das Förderrohr nach unten zum Rohrende hin trichterförmig erweitern. Zum einen ist dadurch die Gefahr, dass sich das Förderrohr zusetzt, reduziert, während andererseits über diesen Trichter auch eine breitere Verteilung beim Einbringen der Biomasse möglich ist, was eine schnellere gleichmäßigere Verteilung der neu zudosierten Biomasse im Fermenterbehälterinnenraum, z. B. in Verbindung mit einem sich im Fermenterbehälterinnenraum angeordneten Rührwerk, ermöglicht.

Gemäß einer alternativen Ausführungsform hierzu ist mit den Merkmalen des Anspruchs 8 eine der Behälter-Auslassöffnung zugeordnete Förder- und Zudosiereinrichtung vorgesehen, mittels der die Biomasse in den Fermenterinnenbereich förderbar ist. Mit einer derartigen Förderung wird auf einfache und funktionssichere Weise sichergestellt, dass eine genau abgestimmte Biomassemenge in den Fermenterinnenbereich in den Feststoffbereich eingedrückt werden kann. Bevorzugt weist die Förder- und Zudosiereinrichtung nach Anspruch 9 hierzu eine Förderschnecke als Zudosier-Schnecke auf, die mit einem Schneckengehäuse gasdicht durch eine Fermenterwand, vorzugsweise die Fermenterbehälterdecke oder eine Behälterseitenwand, geführt ist, wobei wenigstens das Schneckengehäuse mit einem Gehäuseendbereich in die Fermenterflüssigkeit eintaucht. Vorteilhaft wird mit einer derartigen Förderschnecke ein einfaches Eindrücken der neu zudosierten Biomasse in den Bereich der Fermenterflüssigkeit möglich. Durch das Eintauchen wenigstens des Gehäuseendbereichs der Zudosier-Schnecke wird auch hier wiederum auf einfache Weise ohne zusätzliche Abdichtungsmaßnahmen ein Entweichen des sich oberhalb des Flüssigkeitsspiegels ansammelnden Biogases aus dem Fermenterbehälter vermieden. Eine besonders vorteilhafte Anordnung ergibt sich, wenn die Zudosier-Schnecke als Vertikalschnecke in etwa vertikal in den Fermenterbehälter hineinragt. Grundsätzlich sind jedoch auch geneigte Anordnungen der Zudosier-Schnecke möglich, falls dies spezielle Einbaugegebenheiten erfordern.

Ein kompakter und preiswerter Aufbau mit wenig Bauteilaufwand ergibt sich mit den Merkmalen des Anspruchs 10, wenn ein aus dem Fermenterbehälter ragender oberer Schneckengehäusebereich unmittelbar der Behälter-Auslassöffnung zugeordnet ist. Dieser obere Schneckengehäusebereich kann sich hier zur Behälter-Auslassöffnung hin trichterförmig erweitern. Beispielsweise ist hier ein Vorratsbehälter über ein Gestell oberhalb dem aus dem Fermenterbehälter ragenden Schneckengehäuse-Endbereich angeordnet.

Alternativ dazu kann es je nach den baulichen Gegebenheiten aber auch erforderlich sein, dass der Vorratsbehälter und damit die Behälter-Auslassöffnung beabstandet von der Zudosier-Schnecke anzuordnen ist. Hier kann dann die Förder- und Zudosiereinrichtung nach Anspruch 11 eine der Behälter-Auslassöffnung zugeordnete Biomasse-Austragseinrichtung aufweisen, mittels der die von der Behälter-Auslassöffnung freigegebene Biomassemenge zur Zudosier-Schnecke förderbar ist. Grundsätzlich kann anstelle der Zudosier-Schnecke auch eine andere Fördereinrichtung verwendet werden, sofern diese geeignet ist, in der Art und Weise einer Zudosier-Schnecke Biomasse in den Fermenterinnenraum zu fördern. Weiter kann das Schneckengehäuse grundsätzlich verschiedenartigst ausgebildet sein. Das Schneckengehäuse ist bevorzugt schachtförmig und/oder zylindrisch mit eckigem und/oder rundem Querschnitt ausgebildet. Alternativ oder zusätzlich dazu kann die Gehäuseinnenwand wenigstens bereichsweise mit austauschbaren Verschleißplatten ausgekleidet sein. Diese Verschleißplatten können beispielsweise aus Holz und/oder Kunststoff und/oder Blech ausgebildet sein und dienen dazu, die Gehäuseinnenwand vor Beschädigungen durch die Schnecke und/oder Feststoffteile zu schützen. Um die Zudosier-Schnecke stabil am Fermenterbehälter anzuordnen, kann ein Stützgestell vorgesehen sein, mit dem die Zudosier-Schnecke abgestützt und gehalten ist. Dieses Stützgestell ist vorzugsweise im Bereich des Fermenterbehälterdeckels, der durch eine Betondecke oder wenigstens bereichsweise durch ein Foliendach gebildet sein kann, fest verschraubt.

Gemäß Anspruch 12 ist vorgesehen, dass die Austragseinrichtung durch wenigstens ein Förderband und/oder eine Trogschnecke und/oder einen Kratzerförderboden gebildet ist. Diese können z. B. mit einer Messeinrichtung gekoppelt sein oder auch in der Geschwindigkeit regulierbar sein, was z. B. in Verbindung mit einer Austragung einer genau definierten Biomassemenge aus dem Vorratsbehälter von Vorteil sein kann. Je nach dem Grad, mit dem die die Biomasse bildenden organischen Feststoffe mit Flüssigkeiten durchsetzt sind, kann insbesondere eine Trogschnecke, d. h. eine von einem Schneckengehäuse ummantelte Schnecke vorteilhaft sein, da hierdurch sichergestellt ist, dass keine Flüssigkeit auf dem Weg von der Behälter-Auslassöffnung zur Einwurföffnung der Zudosier-Schnecke verloren gehen kann. Auch im umgekehrten Fall, bei einer besonders trockenen und kleingeheckselten Biomasse kann mittels einer Trogschnecke verhindert werden, dass das Biomassematerial z. B. durch den Wind zerstäubt wird.

Die Fermenterbehälter weisen in der Regel einen Durchmesser bis zwanzig Meter und mehr sowie eine Höhe zwischen drei bis acht Meter auf, wobei die Fermenterbehälter üblicherweise im Erdboden eingelassen sind oder als Hochbehälter ausgeführt sind. Je nach der gewählten Ausführungsform, kann dann der Vorratsbehälter z. B. seitlich neben dem Fermenterbehälter angeordnet sein, so dass die Austragseinrichtung von der regelmäßig in einem unteren Vorratsbehälterbereich angeordneten Behälter-Auslassöffnung ausgehend schräg nach oben zu einer Einwurföffnung an der Zudosier-Schnecke geführt ist. Hierdurch ist ferner auch eine einfache und funktionssichere Zuführung der vorbestimmten Biomassemenge aus dem unteren Bereich des Vorratsbehälters in den Schneckenbereich möglich. Unter Umständen wäre hier auch eine Einbringung in einem unteren Fermenterbereich oder einem unteren Schneckenbereich möglich.

Nach Anspruch 13 ist eine Dosiereinrichtung vorgesehen, die die Behälter-Auslassöffnung als Dosieröffnung sowie eine diese verschließende Abdeckeinrichtung aufweist und die vorzugsweise mit einer Steuer- und/oder Regeleinrichtung nach Anspruch 14 gekoppelt ist, die die Abdeckeinrichtung zur Freigabe der Behälter-Auslassöffnung in Abhängigkeit von einem von der Wägeeinrichtung gelieferten Wägesignal so ansteuert, dass eine vorgegebene Biomassenmenge freigegeben wird. Mit einem derartigen Aufbau kann durch ein einfaches Öffnen und Schließen der z. B als Abdeckklappe oder Schieber ausgebildeten Abdeckeinrichtung eine genau definierte Biomassemenge jeweils in Abhängigkeit von dem über die Wägeeinrichtung gelieferten Wägesignal und den vorgegebenen, an den Fermentationsprozess angepassten Parametern freigegeben werden. Damit ist eine hohe Funktionssicherheit beim Betrieb einer derartigen Feststoff-Zudosier-Vorrichtung für eine exakte Zudosierung möglich. Zusätzlich dazu oder aber auch alternativ ist es auch möglich, z. B. eine Austragseinrichtung, die von der Behälter-Auslassöffnung zu einer Zudosier-Schnecke führt, mittels einer Steuer- und/oder Regeleinrichtung so anzusteuern, dass eine vorbestimmte Biomassenmenge mit entsprechend an die auszutragende Biomassemenge angepasster Austragsgeschwindigkeit und/oder Austragszeit ausgetragen wird. Bei einem derartigen Aufbau könnten somit zusätzlich zu dem Wägesignal auch die Geschwindigkeit der Austragseinrichtung sowie die Austragszeit dazu verwendet werden, in Abhängigkeit von einem geeigneten Biomassematerial eine vorgegebene Biomassenmenge auszutragen.

Ein griffiger Transport mit einer vorteilhaften Zerkleinerung von Feststoffen wird mit den Merkmalen des Anspruchs 15 erzielt.

Als Antrieb für die Zudosier-Schnecke ist nach Anspruch 16 ein hydraulischer und/oder elektrischer Antrieb vorgesehen. Weiter kann auch ein Antrieb über eine Kardanwelle eines Vorratsbehälters, der wenigstens eine antreibbare Mischwalze aufweist, vorgesehen sein. Hierzu ist dann lediglich ein Antrieb sowohl für die Mischwalze als auch für die Zudosier-Schnecke erforderlich.

Grundsätzlich kann der Vorratsbehälter in jeder geeigneten Weise stationär am Fermenter angeordnet sein. Gemäß einer weiteren Ausgestaltung nach Anspruch 17 kann der Vorratsbehälter jedoch auch verfahrbar ausgebildet sein, damit der Vorratsbehälter entfernt vom Fermenter mit Biomasse beladen werden kann. Vorzugsweise weist der Vorratsbehälter hierzu Räder auf, damit dieser mittels einer Zugmaschine, z. B. einem Traktor, gezogen werden kann. Dies ist insbesondere dann von Vorteil, wenn Biomasse, beispielsweise Hühnermist, z. B. wegen einer Geruchsbelästigung täglich abgeholt bzw. weggefahren werden muss. Durch die fahrbare Ausbildung kann das Beladen und Zudosieren dann direkt am Ort der angefallenen Biomasse erfolgen, so dass das Zwischenschalten eines weiteren Transportfahrzeugs, was jedes Mal zeit- und arbeitsaufwendig ist und ggf. jedes Mal eine zusätzliche Geruchsbelästigung verursacht, vermieden werden kann. Im Falle von im Vorratsbehälter angeordneten Mischwalzen können diese ggf. auch direkt vom Zugfahrzeug angetrieben werden. Grundsätzlich kann das Verfahren aber auch über Schienen erfolgen, um z. B. den Vorratsbehälter zwischen einer Beladestation und dem Fermenter zu verfahren.

Gemäß einer besonders bevorzugten Ausführungsform nach Anspruch 18 ist im Fermenterbehälter zudem wenigstens ein Rührwerk angeordnet, das vorzugsweise entlang einer Behälterhochachse höhenverstellbar und/oder um diese Behälterhochachse schwenkbar ist. Mit einem derartigen Rührwerk ist eine besonders vorteilhafte und gute Verteilung der neu in den Fermenterbehälter zudosierten Biomasse im Fermenterbehälter möglich, wodurch sich auf besonders vorteilhafte Art und Weise gleichmäßige Fermentationsbedingungen im Fermentationsbehälter auf schnelle Weise erzielen lassen. Dies trägt ebenfalls wesentlich zur Vergleichmäßigung des Fermentationsprozesses bei.

Anhand einer Zeichnung wird die Erfindung näher erläutert:

Es zeigen:
- Fig. 1: einen schematischen Querschnitt durch einen Fermenterbehälter mit einer ersten Ausführungsform einer erfindungsgemäßen Biomasse-Zudosier-Vorrichtung,
- Fig. 2: einen schematischen Querschnitt durch einen Fermenterbehälter mit einer zweiten Ausführungsform einer erfindungsgemäßen Biomasse-Zudosier-Vorrichtung,
- Fig. 3: einen schematischen Querschnitt durch einen Fermenterbehälter mit einer dritten Ausführungsform einer erfindungsgemäßen Biomasse-Zudosier-Vorrichtung,
- Fig. 4: einen schematischen Querschnitt durch einen Fermenterbehälter mit einer vierten Ausführungsform einer erfindungsgemäßen Biomasse-Zudosier-Vorrichtung, und
- Fig. 5: eine schematische Draufsicht auf ein Wendel der Zudosier-Schnecke mit halbmondförmigen Ausnehmungen.

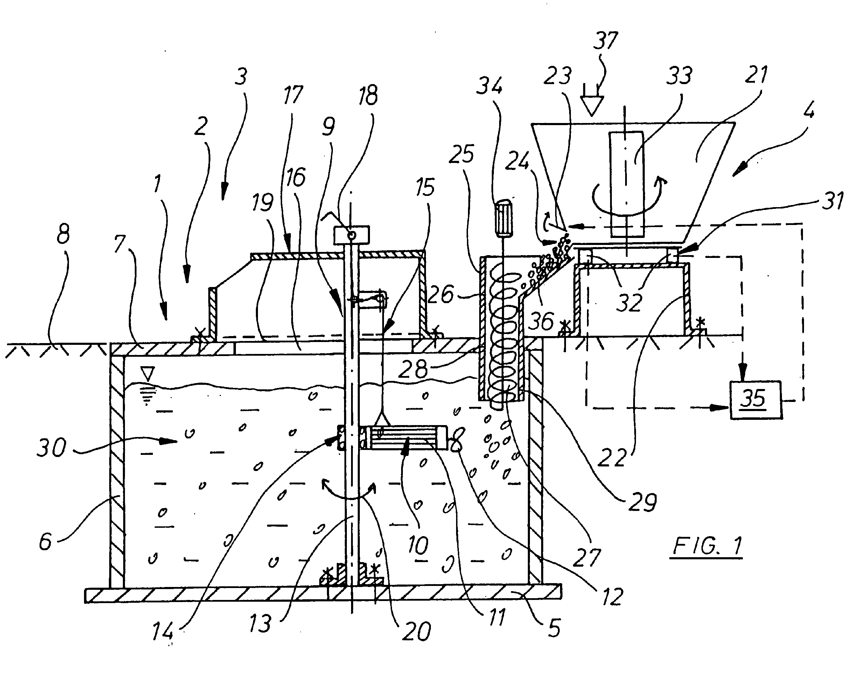

In Fig. 1 ist ein schematischer Querschnitt durch einen Fermenterbehälter 1 eines Fermenters 2 einer Biogasanlage 3 gezeigt mit einer ersten Ausführungsform einer erfindungsgemäßen Biomasse-Zudosier-Vorrichtung 4.

Der Fermenterbehälter 1 weist einen Behälterboden 5, eine vorzugsweise zylindrische Behälterseitenwand 6 sowie einen Behälterdeckel 7 auf. Der Fermenterbehälter 1 ist hier beispielhaft in einem Boden 8 angeordnet, so dass der Behälterdeckel 7 in etwa bodenoberflächenbündig ausgerichtet ist.

Im Fermenterbehälter 1 ist ein Rührwerk 9 vorzugsweise seitlich oder mittig angeordnet, das aus einer Rühreinheit 10, die hier beispielsweise aus einem Tauchmotor 11 und einem Rührer aufgebaut ist, und einer im Fermenterbehälter 1 vertikal gehaltenen Stützstange 13 besteht, wobei die Rühreinheit 10 über eine Führungseinrichtung 14, die hier als Wälzlagerführung ausgebildet ist, höhenverstellbar an der Stützstange 13 gehalten ist. Zur Höhenverstellung der Rühreinheit 10 ist eine Betätigungseinrichtung 15, die hier beispielsweise durch eine Seilzuganordnung als kraftübertragende Verbindung ausgebildet ist, vorgesehen. Im Behälterdeckel 7 ist eine Rührwerköffnung 16 ausgebildet, durch die die Stützstange 13 und die Rühreinheit 10 in den Fermenterbehälter 1 einführbar sind, wobei über der Rührwerköffnung 16 ein Dom 17 als gasdichte Abdeckung angebracht ist und der obere Stützstangenbereich mit einer Handkurbel 18 der Betätigungseinrichtung über eine Dachwand des Doms 17 ragt. Der Dom 17 kann über eine hier nicht näher dargestellte Tür oder Klappe betreten werden, so dass die mittels der Betätigungseinrichtung 15 in den Dom 17 bewegte Rühreinheit im Innenraum oder von außerhalb des Doms 17 zugänglich ist. Die Rührwerköffnung 16 ist, wie dies in der Fig. 1 lediglich strichliert angedeutet ist in diesem Falle mittels einer Abdeckung 19 gasdicht abdeckbar. Wie dies in der Fig. 1 durch den Pfeil 20 angedeutet ist, kann die Rühreinheit 10 mittels der Stützstange 13 im Fermenterbehälterinnenraum entlang der Stützstangenlängsachse verschwenkt werden.

Wie dies aus der Fig. 1 weiter entnommen werden kann, ist seitlich neben dem Behälterdeckel 7 ein Vorratsbehälter 21 angeordnet, der auf einem Stützgestell 22 im Abstand vom Erdboden 8 angeordnet ist. An diesem Vorratsbehälter 21 ist eine mittels einer hier lediglich schematisch als Abdeckklappe dargestellten Abdeckeinrichtung 23 verschließbare Behälter-Auslassöffnung 24 ausgebildet, der ein zur Behälter-Auslassöffnung 24 hin trichterförmig erweiterter oberer Schneckengehäusebereich 25 eines Schneckengehäuses 26 einer als Förderschnecke ausgebildeten Zudosier-Schnecke 27 zugeordnet ist, die in der Darstellung der Fig. 1 als Vertikal-Schnecke gasdicht und vertikal durch eine im Behälterdeckel 7 ausgebildete Fermenterzuführöffnung 28 geführt ist, wobei das Schneckengehäuse 26 mit einem unteren Gehäuseendbereich 29 in die Fermenterflüssigkeit 30 eintaucht.

Der Vorratsbehälter 21 ist mit einer Wägeeinrichtung 31 gekoppelt, die hier lediglich beispielhaft durch vier Wägestäbe 32 gebildet ist, von denen in der Darstellung der Fig. 1 lediglich zwei gezeigt sind und die jeweils einem Eckbereich einer in etwa rechteckförmigen Bodenplatte des Vorratsbehälters 21 zugeordnet sind, sowie auf denen der Vorratsbehälter 21 in wägetechnisch üblicher Weise gelagert ist.

Im Vorratsbehälter 21 sind hier beispielsweise zusätzlich zwei vertikal ausgerichtete Zerkleinerungs- und Mischwalzen 33 angeordnet, von denen lediglich eine gezeigt ist.

Die Zudosier-Schnecke 27 ist mittels einem Elektromotor 34 antreibbar. Ebenso sind auch die vertikalen Zerkleinerungs- und Mischwalzen 33 über einen hier nicht dargestellten Elektromotor antreibbar. Auch ein Hydraulik- und/oder Wasser- und/oder Luftantrieb wäre hier möglich.

Im konkreten Einsatzfall kann die großvolumige Zudosier-Schnecke 27 beispielsweise einen Durchmesser von 300 bis 600 Millimeter aufweisen. Die Eintauchtiefe des Gehäuseendbereichs 29 des Schneckengehäuses 26 und ggf. auch der Zudosier-Schnecke 27 in die Fermenterflüssigkeit 30 kann dabei je nach Bedarf bis zu ca. 500 Millimeter betragen.

Wie dies aus der schematischen Darstellung der Fig. 1 weiter ersichtlich ist, sind die Wägestäbe 32 mit einer Steuer- und Regeleinrichtung 35 gekoppelt, die die Abdeckeinrichtung 23 zur Freigabe der Behälter-Auslassöffnung 24 in Abhängigkeit von einem von den Wägestäben 32 gelieferten Wägesignals so ansteuert, dass eine vorgegebene Menge an Biomasse 36 freigegeben wird, die dann über die trichterförmige Erweiterung des oberen Schneckengehäusebereichs 25 in den Bereich der Zudosier-Schnecke 27 rutscht und anschließend von der Zudosier-Schnecke 27 unterhalb des Flüssigkeitsspiegels in die Fermenterflüssigkeit 30 eingedrückt wird. Mittels der Rühreinheit 10 kann dann die neu zudosierte Biomasse 36 im Fermenterbehälter 1 gleichmäßig verteilt werden. Nachdem die vorgegebene Menge an Biomasse 36 aus dem Vorratsbehälter 21 ausgegeben ist, wird die Abdeckeinrichtung 23 wieder geschlossen.

Wie dies in der Fig. 1 durch den Pfeil 37 schematisch angedeutet ist, können in den Vorratsbehälter 21 verschiedenste organische Feststoffe, wie z. B. Silomais, Mist, Kartoffeln oder dergleichen, die mehr oder weniger mit Flüssigkeit vermischt sein können, aufgrund der Wägeeinrichtung 31 mit exakt vorgegebener Menge zudosiert werden, so dass in den Vorratsbehälter 21 ein genau definierter Biomasse-Substratmix eingebracht werden kann.

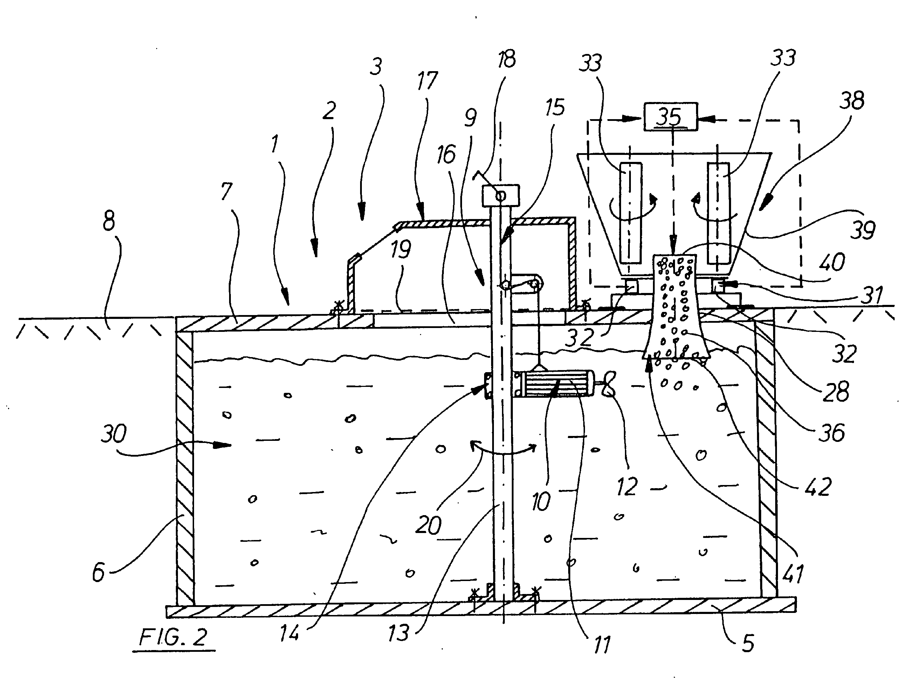

In der Fig. 2 ist eine alternative Ausführungsform einer Biomasse-Zudosier-Vorrichtung 38 gezeigt, die einen grundsätzlich gleichen Aufbau des Fermenterbehälters 1 zeigt, so dass gleiche Teile mit gleichen Bezugszeichen bezeichnet werden. Die Biomasse-Zudosier-Vorrichtung 38 umfasst einen Vorratsbehälter 39, der im unteren Behälterbereich eine verschließbare Behälter-auslassöffnung 40 aufweist. Der Vorratsbehälter 39 ist hier unmittelbar oberhalb der Fermenterzuführöffnung 28 angeordnet, wobei von der Behälter-Auslassöffnung 40 ausgehend ein Förderrohr 41 gasdicht durch den Behälterdeckel 7 geführt und mit einem sich trichterförmig nach unten erweiternden Rohrende 42 in die Fermenterflüssigkeit 30 eintaucht. Auch hier kann somit wieder bei einem Ansteuern der hier nicht gezeigten Verschließ- bzw. Abdeckeinrichtung mittels der Steuer- und Regeleinrichtung 35 in Abhängigkeit von einem Wägesignal der Wägestäbe 32 der Wägeeinrichtung 31 eine genau auf den Fermentationsprozess abgestimmte Biomassenmenge chargenweise in den Fermenterbehälter eingebracht werden.

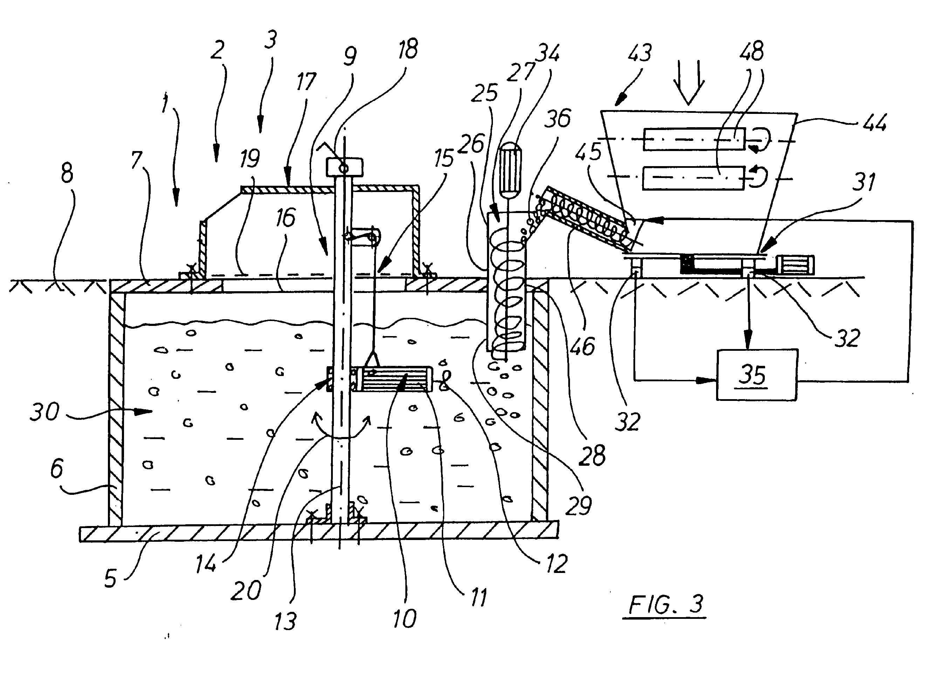

In der Fig. 3 ist eine weitere alternative Ausführungsform einer Biomasse-Zudosier-Vorrichtung 43 gezeigt, bei der ein Vorratsbehälter 44 seitlich neben einem Fermenterbehälter 1 auf dem Boden 8 angeordnet ist und eine Behälter-Auslassöffnung einen Abstand zur Zudosier-Schnecke 27 aufweist. Zur Überbrückung dieses Abstandes ist zwischen der Behälter-Auslassöffnung 45 und der oberen Schneckengehäuse-Erweiterung des Schneckengehäuses 26 eine als Trogschnecke 46 ausgebildete Austragseinrichtung vorgesehen, mit der die vorgegebene Biomassenmenge in den Bereich der Zudosier-Schnecke 27 gefördert wird. Alternativ zu den Aufbauten der Figuren 1 und 2 sind hier die Zerkleinerungs- und Mischwalzen 48 horizontal angeordnet. Ansonsten entspricht der Aufbau demjenigen der Fig. 1, so dass hierauf nicht mehr näher eingegangen wird.

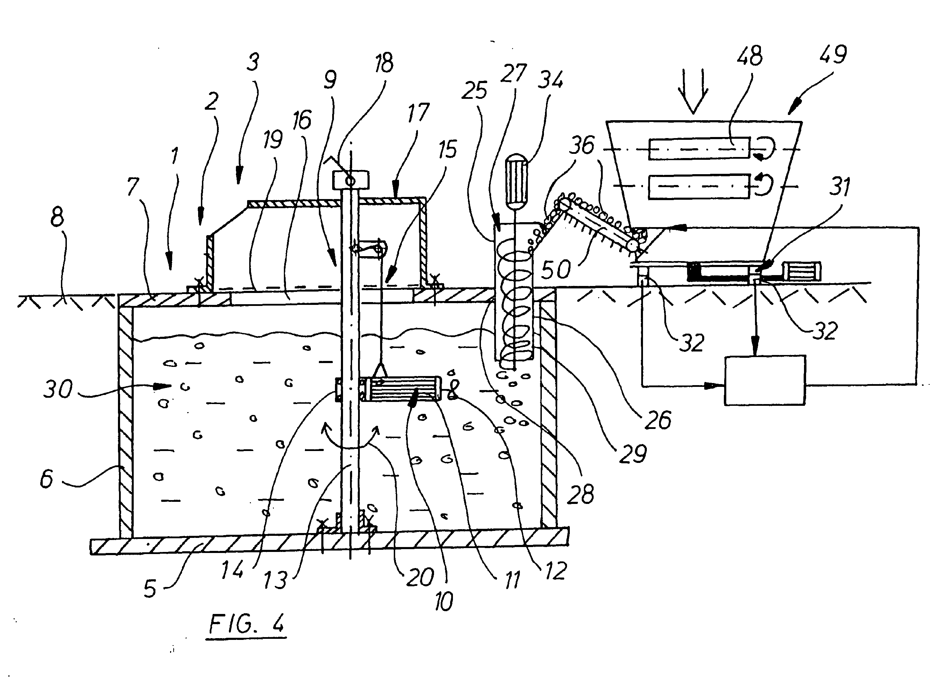

In der Fig. 4 ist eine zur Fig. 3 alternative Ausführungsform dargestellt, bei der eine Biomasse-Zudosier-Vorrichtung 49 vorgesehen ist, bei der die Austragseinrichtung anstelle einer Trogschnecke durch ein Förderband 50 gebildet ist. Ansonsten entspricht der Aufbau demjenigen der Fig. 3, so dass hierauf nicht mehr näher eingegangen wird.

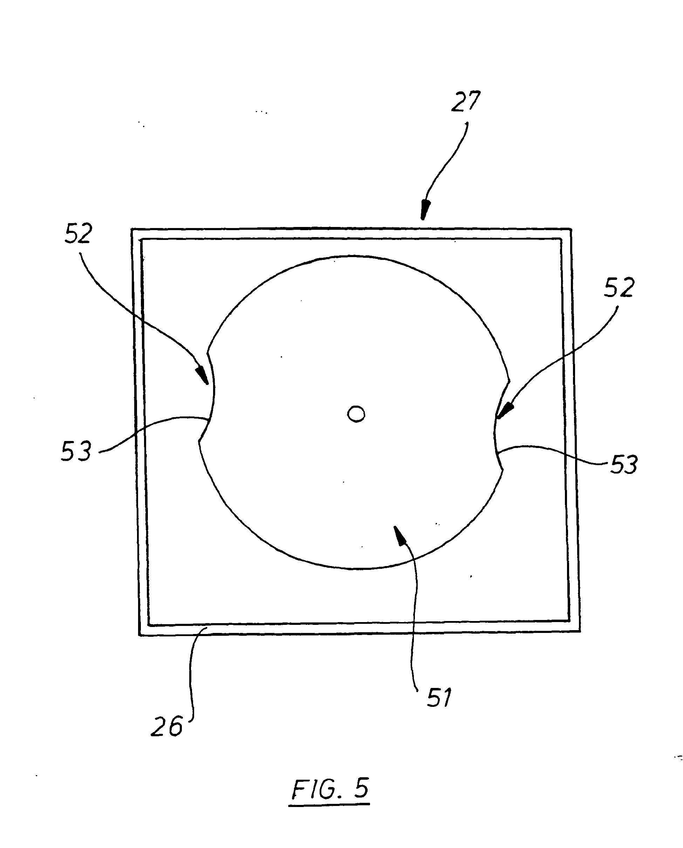

Wie dies aus der Fig. 5 ersichtlich ist, können an den Außenseiten der Schneckenwendel 51 abschnittsweise halbmondförmige Ausnehmungen 52 zur Ausbildung von scharfkantigen Reiß- und Schnittflächen 53 ausgebildet sein.

## Patentansprüche

1. Biomassefeststoff-Zudosier-Vorrichtung an einem Fermenter einer Biogasanlage,
mit einem Fermenterbehälter(1), der wenigstens eine Fermenterzuführöffnung aufweist, über die mittels einer Förderschnecke (27), die mit einem Schneckengehäuse (26) gasdicht durch eine Fermenterbehälterwand (7) geführt ist, organische Feststoffe als Biomasse in den Fermenterbehälter (1) eingebracht werden,
**dadurch gekennzeichnet,**
**dass** die Biomassefeststoff-Zudosier-Vorrichtung (4; 38; 43; 49) ferner eine der Förderschnecke (27) vorgeschaltete Wägeeinrichtung (31) als Gewichtsmesseinrichtung aufweist, mit der zur Vermeidung eines Absackens und/oder Aufschwimmens der Biomassefeststoffe im Fermenterbehälter (1) eine genau auf den Fermentationsprozess im Fermenterbehälterinneren abgestimmte Biomassefeststoffmenge abgewogen und mittels der Förderschnecke (27) chargenweise in den Fermenterbehälter (1) eingebracht wird.

2. Biomasse-Zudosier-Vorrichtung nach Anspruch 1, **dadurch gekennzeichnet, dass** die Wägeeinrichtung in Verbindung mit einem Förderband vorgesehen ist.

3. Biomasse-Zudosier-Vorrichtung nach Anspruch 1, **dadurch gekennzeichnet,**
**dass** ein Vorratsbehälter (21; 39; 44) vorgesehen ist, in dem die Biomasse aufgenommen ist, und
**dass** eine Wägeeinrichtung (31) mit dem Vorratsbehälter (21; 39; 44) gekoppelt ist.

4. Biomasse-Zudosier-Vorrichtung nach Anspruch 3, **dadurch gekennzeichnet, dass** die Wägeeinrichtung (31) durch wenigstens einen Wägestab (32) gebildet ist, auf dem der Vorratsbehälter (21; 39; 44) gelagert ist und der vorzugsweise integral mit dem Vorratsbehälter (21; 39; 44) ausgebildet ist.

5. Biomasse-Zudosier-Vorrichtung nach Anspruch 4, **dadurch gekennzeichnet, dass** zwei oder mehr Wägestäbe (32) vorgesehen sind, mit denen bei ungleichmäßiger Biomasseverteilung im Vorratsbehälter (21; 39; 44) ein gemittelter Biomasse-Gewichtswert ermittelbar ist.

6. Biomasse-Zudosier-Vorrichtung nach einem der Ansprüche 3 bis 5, **dadurch gekennzeichnet, dass** der Vorratsbehälter (21; 39; 44) wenigstens eine verschließbare Behälter-Auslassöffnung (24; 40; 45) aufweist, über die im geöffneten Zustand eine vorzugsweise genau abgestimmte Biomassemenge freigebbar und über die Fermenterzuführöffnung (28) in den Fermenterbehälter (1) einbringbar ist.

7. Biomasse-Zudosier-Vorrichtung nach Anspruch 6, **dadurch gekennzeichnet,**
**dass** über die Behälter-Auslassöffnung (40) eine genau abgestimmte Biomassemenge freigebbar ist,
**dass** von der Behälter-Auslassöffnung (40) ausgehend ein Förderrohr (41) gasdicht durch die in einer Fermenterbehälterwand (7) ausgebildete Fermenterzuführöffnung (28) geführt und mit einem Rohrende (42) in die Fermenterflüssigkeit (30) eintaucht, und
**dass** sich das Förderrohr (41) vorzugsweise nach unten zum Rohrende (42) hin trichterförmig erweitert.

8. Biomasse-Zudosier-Vorrichtung nach Anspruch 6, **dadurch gekennzeichnet, dass** von der Behälter-Auslassöffnung (24; 45) ausgehend eine Förder- und Zudosiereinrichtung (27; 27, 46; 27, 50) gasdicht durch die Fermenterzuführöffnung (28) in den Fermenterinnenbereich geführt ist dergestalt, dass eine genau abgestimmte Biomassemenge mit der Förder- und Zudosiereinrichtung (27; 27, 46; 27, 50) in den Fermenterinnenbereich förderbar ist.

9. Biomasse-Zudosier-Vorrichtung nach Anspruch 8, **dadurch gekennzeichnet,**
**dass** die Förder- und Zudosiereinrichtung eine Förderschnecke als Zudosier-Schnecke (27) aufweist, die mit einem Schneckengehäuse (26) gasdicht sowie vorzugsweise in etwa vertikal durch die Fermenterbehälterwand (7), vorzugsweise die Fermenterbehälterdecke, geführt ist, und
**dass** wenigstens das Schneckengehäuse (26) mit einem Gehäuseendbereich (29) in die Fermenterflüssigkeit eintaucht.

10. Biomasse-Zudosier-Vorrichtung nach Anspruch 9, **dadurch gekennzeichnet,**
**dass** ein aus dem Fermenterbehälter (1) ragender oberer Schneckengehäusebereich (25) unmittelbar der Behälter-Auslassöffnung (24) zugeordnet ist und sich vorzugsweise zur Behälter-Auslassöffnung (24) hin trichterförmig erweitert, und
**dass** über die Behälter-Auslassöffnung (24) eine genau abgestimmte Biomassemenge freigebbar ist.

11. Biomasse-Zudosier-Vorrichtung nach Anspruch 9, **dadurch gekennzeichnet, dass** die Förder- und Zudosiereinrichtung (27, 46; 27, 50) ferner eine der Behälter-Auslassöffnung (45) zugeordnete Biomasse-Austragseinrichtung (46; 50) aufweist, mittels der die von der Behälter-Auslassöffnung (45) freigegebene Biomassemenge zur Zudosier-Schnecke (27) förderbar ist.

12. Biomasse-Zudosier-Vorrichtung nach Anspruch 11, **dadurch gekennzeichnet,**
**dass** die Austragseinrichtung durch wenigstens ein Förderband (50) und/oder eine Trogschnecke (46) und/oder einen Kratzerförderboden gebildet ist, die ggfs. mit einer Messeinrichtung gekoppelt und/oder in der Geschwindigkeit regulierbar sind, und/oder
**dass** der Vorratsbehälter (44) seitlich neben dem vorzugsweise im Boden angeordneten Fermenterbehälter (1) angeordnet ist, so dass die Austragseinrichtung (46; 50) von der in einem unteren Vorratsbehälterbereich angeordneten Behälter-Auslassöffnung (45) ausgehend schräg nach oben zu einer Einwurföffnung an der Zudosier-Schnecke (27) geführt ist.

13. Biomasse-Zudosier-Vorrichtung nach einem der Ansprüche 1 bis 12, **dadurch gekennzeichnet, dass** eine Dosiereinrichtung vorgesehen ist, die die Behälter-Auslassöffnung (24; 40; 45) als Dosieröffnung sowie eine diese verschließende Abdeckeinrichtung (23) aufweist.

14. Biomasse-Zudosier-Vorrichtung nach einem der Ansprüche 1 bis 13, **dadurch gekennzeichnet, dass** die Biomasse-Zudosier-Vorrichtung (4; 38; 43; 49) mit einer Steuer- und/oder Regeleinrichtung (35) gekoppelt ist, die die entsprechenden Bauteile, vorzugsweise die Abdeckeinrichtung (23) zur Freigabe der Behälter-Auslassöffnung (24; 40; 45) in Abhängigkeit von einem von der Wägeeinrichtung (31) gelieferten Wägesignal, so ansteuert und/oder so regelt, dass eine vorgegebene Biomassemenge in den Fermenterbehälter (1) einbringbar ist.

15. Biomasse-Zudosier-Vorrichtung nach einem der Ansprüche 9 bis 14, **dadurch gekennzeichnet, dass** an den Außenseiten der Schneckenwendel (51) der Zudosier-Schnecke (27) wenigstens abschnittsweise Ausnehmungen (52), vorzugsweise halbmondförmige Ausnehmungen (52), zur Ausbildung von scharfkantigen Reiß- und Schnittflächen (53) ausgebildet sind.

16. Biomasse-Zudosier-Vorrichtung nach einem der Ansprüche 9 bis 15, **dadurch gekennzeichnet, dass** die Zudosier-Schnecke (27) hydraulisch und/oder elektrisch antreibbar ist.

17. Biomasse-Zudosier-Vorrichtung nach einem der Ansprüche 1 bis 16, **dadurch gekennzeichnet, dass** der Vorratsbehälter verfahrbar ausgebildet ist, vorzugsweise mit Rädern zum Ziehen mittels einer Zugmaschine.

18. Biomasse-Zudosier-Vorrichtung nach einem der Ansprüche 1 bis 17, **dadurch gekennzeichnet, dass** im Fermenterbehälter (1) wenigstens ein Rührwerk angeordnet ist, das vorzugsweise entlang einer Behälterhochachse höhenverstellbar und/oder um diese schwenkbar ist.

19. Verfahren zur Einbringung von Biomasse in einen Fermenterbehälter (1) einer Biogasanlage mittels einer Biomasse-Zudosier-Vorrichtung (4; 38; 43; 49) nach einem der Ansprüche 1 bis 18.

## Claims

1. Apparatus for supplying biomass solids to a fermenter of a biogas installation,
having a fermenter tank (1) which has at least one fermenter-feed opening via which organic solids are introduced, as biomass, into the fermenter tank (1) by means of a conveying screw (27), which has a screw housing (26) guided in a gas-tight manner through a fermenter-tank wall (7),
**characterized**
**in that** the apparatus (4; 38; 43; 49) for supplying biomass solids also has a weighing arrangement (31), as a weight-measuring arrangement, which is arranged upstream of the conveying screw (27) and by means of which, in order to avoid the biomass solids sinking down and/or floating in the fermenter tank (1), a quantity of biomass solids which is coordinated precisely for the fermentation process in the fermenter-tank interior is weighed and introduced in batches, by means of the conveying screw (27), into the fermenter tank (1).

2. Biomass-supplying apparatus according to Claim 1, **characterized in that** the weighing arrangement is provided in conjunction with a conveying belt.

3. Biomass-supplying apparatus according to Claim 1,
**characterized**
**by** the provision of a storage tank (21; 39; 44), in which the biomass is accommodated, and
by a weighing arrangement (31) being coupled to the storage tank (21; 39; 44).

4. Biomass-supplying apparatus according to Claim 3, **characterized in that** the weighing arrangement (31) is formed by at least one weighing bar (32) on which the storage tank (21; 39; 44) is mounted and which is formed preferably integrally with the storage tank (21; 39; 44).

5. Biomass-supplying apparatus according to Claim 4, **characterized by** the provision of two or more weighing bars (32) which, in the case of a non-uniform distribution of biomass in the storage tank (21; 39; 44), make it possible to determine an average biomass-weight value.

6. Biomass-supplying apparatus according to one of Claims 3 to 5, **characterized in that** the storage tank (21; 39; 44) has at least one closable tank-outlet opening (24; 40; 45) via which, in the open state, a preferably precisely coordinated quantity of biomass can be released and can be introduced, via the fermenter-feed opening (28), into the fermenter tank (1).

7. Biomass-supplying apparatus according to Claim 6,
**characterized**
**in that** a precisely coordinated quantity of biomass can be released via the tank-outlet opening (40),
**in that**, starting from the tank-outlet opening (40), a conveying pipe (41) is guided in a gas-tight manner through the fermenter-feed opening (28), formed in a fermenter-tank wall (7), and has a pipe end (42) penetrating into the fermenter liquid (30), and
**in that** the conveying pipe (41) preferably widens downwards, in the direction of the pipe end (42), in a hopper-like manner.

8. Biomass-supplying apparatus according to Claim 6, **characterized in that**, starting from the tank-outlet opening (24; 45), a conveying and supplying arrangement (27; 27, 46; 27, 50) is guided in a gas-tight manner into the inner region of the fermenter, through the fermenter-feed opening (28), such that a precisely coordinated quantity of biomass can be conveyed into the inner region of the fermenter by the conveying and supplying arrangement (27; 27, 46; 27, 50).

9. Biomass-supplying apparatus according to Claim 8,
**characterized**
**in that** the conveying and supplying arrangement has a conveying screw as supplying screw (27), which has a screw housing (26) guided in a gas-tight manner, and preferably approximately vertically, through the fermenter-tank wall (7), preferably the fermenter-tank top, and
**in that** at least the screw housing (26) has a housing end region (29) penetrating into the fermenter liquid.

10. Biomass-supplying apparatus according to Claim 9,
**characterized**
**in that** a top screw-housing region (25), which projects out of the fermenter tank (1), is assigned directly to the tank-outlet opening (24) and widens in a hopper-like manner preferably in the direction of the tank-outlet opening (24), and
**in that** a precisely coordinated quantity of biomass can be released via the tank-outlet opening (24).

11. Biomass-supplying apparatus according to Claim 9, **characterized in that** the conveying and supplying arrangement (27, 46; 27, 50) also has a biomass-discharging arrangement (46; 50) which is assigned to the tank-outlet opening (45) and by means of which the quantity of biomass released from the tank-outlet opening (45) can be conveyed to the supplying screw (27).

12. Biomass-supplying apparatus according to Claim 11,
**characterized**
**in that** the discharging arrangement is formed by at least one conveying belt (50) and/or a trough-type screw (46) and/or a scraper conveyor, which are coupled, if appropriate, to a measuring arrangement and/or can be regulated in speed, and/or
**in that** the storage tank (44) is arranged laterally alongside the fermenter tank (1), which is preferably arranged in the ground, in which case the discharging arrangement (46; 50) is guided obliquely upwards, starting from the tank-outlet opening (45) arranged in a bottom storage-tank region, to an introduction opening at the supplying screw (27).

13. Biomass-supplying apparatus according to one of Claims 1 to 12, **characterized by** the provision of a metering arrangement which has the tank-outlet opening (24; 40; 45) as metering opening and also a covering arrangement (23), which closes this opening.

14. Biomass-supplying apparatus according to one of Claims 1 to 13, **characterized in that** the biomass-supplying apparatus (4; 38; 43; 49) is coupled to a control and/or regulating arrangement (35) which activates and/or regulates the corresponding components, preferably the covering arrangement (23) for releasing the tank-outlet opening (24; 40; 45) in dependence on a weighing signal supplied by the weighing arrangement (31), such that a predetermined quantity of biomass can be introduced into the fermenter tank (1).

15. Biomass-supplying apparatus according to one of Claims 9 to 14, **characterized in that** recesses (52), preferably half-moon-shaped recesses (52), are formed, at least in certain sections, on the outer sides of the screw helix (51) of the supplying screw (27) in order to form sharp-edged tearing and cutting surfaces (53).

16. Biomass-supplying apparatus according to one of Claims 9 to 15, **characterized in that** the supplying screw (27) can be driven hydraulically and/or electrically.

17. Biomass-supplying apparatus according to one of Claims 1 to 16, **characterized in that** the storage tank is of mobile design, preferably with wheels in order to be pulled by means of a tractor.

18. Biomass-supplying apparatus according to one of Claims 1 to 17, **characterized in that** the fermenter tank (1) contains at least one agitator which can be adjusted vertically preferably along a vertical tank axis and/or can be pivoted about the latter.

19. Method of introducing biomass into a fermenter tank (1) of a biogas installation by means of a biomass-supplying apparatus (4; 38; 43; 49) according to one of Claims 1 to 18.

## Revendications

1. Dispositif de dosage de biomasse solide sur un fermentateur d'une installation de biogaz,
avec un conteneur de fermentateur (1), qui présente au moins une ouverture d'alimentation de fermenteur, par le biais de laquelle des substances solides organiques sont introduites en tant que biomasse dans le conteneur de fermentateur (1) au moyen d'une vis sans fin (27), qui est guidée avec un carter de la vis sans fin (26) de manière étanche au gaz à travers une paroi de conteneur de fermentateur (7),
**caractérisé en ce**
**que** le dispositif de dosage de biomasse solide (4; 38 ; 43 ; 49) présente en outre en tant que dispositif de mesure du poids un dispositif de pesée (31) placé en amont de la vis sans fin (27), avec lequel une quantité de biomasse solide exactement adaptée au processus de fermentation à l'intérieur du conteneur de fermentateur est pesée pour éviter un enfoncement et/ou un flottement de la biomasse solide dans le conteneur de fermentateur (1) et est introduite par charges successives dans le conteneur de fermentateur (1) au moyen de la vis sans fin (27).

2. Dispositif de dosage de biomasse selon la revendication 1, **caractérisé en ce que** le dispositif de pesée est prévu en relation avec un transporteur.

3. Dispositif de dosage de biomasse selon la revendication 1, **caractérisé en ce**
**qu'**un réservoir (21; 39; 44) est prévu, qui reçoit la biomasse, et
en ce qu'un dispositif de pesée (31) est couplé au réservoir (21 ; 39 ; 44).

4. Dispositif de dosage de biomasse selon la revendication 3, **caractérisé en ce que** le dispositif de pesée (31) est formé par au moins une tige de pesée (32) sur laquelle est logé le réservoir (21 ; 39 ; 44) et qui est réalisé de préférence de manière intégrale avec le réservoir (21 ; 39 ; 44).

5. Dispositif de dosage de biomasse selon la revendication 4, **caractérisé en ce que**, deux ou plusieurs tiges de pesée (32) sont prévues avec lesquelles on peut déterminer une valeur de poids de biomasse moyenne en cas de répartition inégale de la biomasse dans le réservoir (21 ; 39 ; 44).

6. Dispositif de dosage de biomasse selon l'une quelconque des revendications 3 à 5, **caractérisé en ce que** le réservoir (21 ; 39 ; 44) présente au moins une ouverture d'échappement de conteneur pouvant être fermée (24 ; 40 ; 45), par le biais de laquelle, à l'état ouvert, une quantité de biomasse de préférence exactement accordée peut être délivrée et introduite dans le conteneur de fermentateur (1) par le biais de l'ouverture d'alimentation de fermentateur (28).

7. Dispositif de dosage de biomasse selon la revendication 6, **caractérisé en ce**
**qu'**une quantité de biomasse exactement accordée peut être libérée par le biais de l'ouverture d'échappement de conteneur (40),
en ce qu'un tube de transport (41) partant de l'ouverture d'échappement de conteneur (40) est amené de manière étanche au gaz à travers l'ouverture d'alimentation de fermentateur (28) réalisée dans la paroi de conteneur de fermentateur (7) et plonge dans le liquide de fermentateur (30) avec une extrémité de tube (42), et
en ce que le tube de transport (41) s'élargit de préférence vers le bas en une extrémité de tuyau (42) en forme d'entonnoir.

8. Dispositif de dosage de biomasse selon la revendication 6, **caractérisé en ce qu'**un dispositif de dosage et de transport (27 ; 27, 46 ; 27, 50) partant de l'ouverture d'échappement de conteneur (24 ; 45) est amené de manière étanche au gaz à travers l'ouverture d'alimentation de fermentateur (28) dans la zone intérieure du fermentateur, à tel point qu'une quantité de biomasse exactement accordée peut être acheminée avec le dispositif de dosage et de transport (27 ; 27, 46 ; 27, 50) dans la zone intérieure du fermentateur.

9. Dispositif de dosage de biomasse selon la revendication 8, **caractérisé en ce**
**que** le dispositif de dosage et de transport présente une vis sans fin de transport en tant que vis sans fin de dosage (27), qui est amenée avec un carter de vis en fin (26) de manière étanche au gaz ainsi que de préférence de manière à peu près verticale à travers la paroi du conteneur de fermentateur (7), de préférence la partie supérieure du conteneur de fermentateur et
en ce qu'au moins le carter de vis en fin (26) plonge dans le liquide de fermentateur avec une zone d'extrémité de carter (29).

10. Dispositif de dosage de biomasse selon la revendication 9, **caractérisé en ce**
**qu'**une zone de carter de vis sans fin (25) supérieure dépassant du conteneur de fermentateur (1) est associé directement à l'ouverture d'échappement de conteneur (24) et s'élargit de préférence en une ouverture d'échappement de conteneur (24) en forme d'entonnoir, et
en ce qu'une quantité de biomasse exactement accordée peut être libérée par le biais de l'ouverture d'échappement de conteneur (24).

11. Dispositif de dosage de biomasse selon la revendication 9, **caractérisé en ce que** le dispositif de dosage et de transport (27, 46 ; 27, 50) présente en outre un dispositif de sortie de biomasse (46 ; 50) associé à l'ouverture d'échappement de conteneur (45), au moyen duquel la quantité de biomasse délivrée par l'ouverture d'échappement de conteneur (45) peut être acheminée à la vis de dosage (27).

12. Dispositif de dosage de biomasse selon la revendication 11, **caractérisé en ce**
**que** le dispositif de sortie est formé par au moins un transporteur (50) et/ou une vis sans fin à auges (46) et/ou un fond d'entraîneur racleur, qui sont couplés le cas échéant à un dispositif de mesure et/ou réglables en vitesse, et/ou
en ce que le réservoir (44) est disposé de manière latérale à côté du conteneur de fermentateur (1) disposé de préférence dans le sol, de sorte que le dispositif de sortie (46 ; 50) est amené en biais vers le haut en partant de l'ouverture d'échappement de conteneur (45) disposée dans une zone inférieure du réservoir à une ouverture d'introduction au niveau de la vis sans fin de dosage (27).

13. Dispositif de dosage de biomasse selon l'une quelconque des revendications 1 à 12, **caractérisé en ce qu'**un dispositif de dosage est prévu, qui présente l'ouverture d'échappement de conteneur (24 ; 40 ; 45) en tant qu'ouverture de dosage ainsi qu'un dispositif de recouvrement d'obturation (23).

14. Dispositif de dosage de biomasse selon l'une quelconque des revendications 1 à 13, **caractérisé en ce que** le dispositif de dosage de biomasse (4 ; 38 ; 43 ; 49) est couplé à un dispositif de commande et/ou de régulation (35), qui commande et/ou règle les pièces correspondantes, de préférence le dispositif de recouvrement (23) permettant la libération de l'ouverture d'échappement de conteneur (24 ; 40 ; 45) en fonction d'un signal de poids délivré par le dispositif de pesée (31), de telle sorte qu'une quantité de biomasse prédéfinie peut être introduite dans le conteneur de fermentateur (1).

15. Dispositif de dosage de biomasse selon l'une quelconque des revendications 9 à 14, **caractérisé en ce que** des évidements (52) sont formés au moins par section sur les côtés extérieurs de l'hélice de la vis sans fin (51) de la vis sans fin de dosage (27), de préférence des évidements en forme de demi-lune (52), pour former des surfaces de déchirure et de coupe à arêtes vives (53).

16. Dispositif de dosage de biomasse selon l'une quelconque des revendications 9 à 15, **caractérisé en ce que** la vis sans fin de dosage (27) peut être entraînée de manière hydraulique et/ou électrique.

17. Dispositif de dosage de biomasse selon l'une quelconque des revendications 1 à 16, **caractérisé en ce que** le réservoir est réalisé de manière à pouvoir être déplacé, de préférence avec des roues pour être tiré au moyen d'une machine de traction.

18. Dispositif de dosage de biomasse selon l'une quelconque des revendications 1 à 17, **caractérisé en ce qu'**au moins un mélangeur est disposé dans le conteneur de fermentateur (1), dont la hauteur est réglable le long de l'axe vertical du conteneur et/ou qui peut pivoter autour de celui-ci.

19. Procédé d'introduction de biomasse dans un conteneur de fermentateur (1) d'une installation de biomasse au moyen d'un dispositif de dosage de biomasse (4 ; 38 ; 43 ; 49) selon l'une quelconque des revendications 1 à 18.
